# EUROPEAN PATENT APPLICATION

(11) **EP 2 468 109 A1**
(43) Date of publication of application: **27.06.2012**
(21) Application number: 10196255.3
(22) Date of filing: 21.12.2010
(51) Int. Cl.: A23J 3/34, A23J 1/14, A23L 1/305

(54) **Plant protein hydrolysates**

(71) Applicant: Nestec S.A., 1800 Vevey (CH)
(72) Inventor: Berends, Pieter,, 78357 Zoznegg-Mühlingen, (DE); Fischer, Lutz,, 70186 Stuttgart, (DE); Linke, Diana,, 31547 Bad Rehburg, (DE); Rabe, Swen,, 78357 Mühlingen, (DE); Berger, Ralf Gunter,, 30455 Hannover, (DE)

(57) **Abstract**

Plant protein hydrolysates, an apparatus and a method for the manufacture thereof are disclosed. The apparatus comprises an ultra filtration module upon which the plant protein hydrolysates pass through allowing a reuse of an enzyme source.

## Description

### Cross reference to related applications

None.

### Field Of Invention

The present invention relates to a hydrolysis of plant proteins to form plant protein hydrolysates. In particular the present invention relates an apparatus and a use of the apparatus for the manufacture of the plant protein hydrolysates. The invention also relates to a method for the manufacture of the plant protein hydrolysates.

### Background of invention

Protein hydrolysates such as amino acids and peptides have applications in food technology. The protein hydrolysates are used for providing taste active ingredients to food products.

Protein hydrolysates are manufactured by hydrolysis of a protein. Protein hydrolysates can therefore include amino acids and peptides which are obtained by the hydrolysis of the protein.

A use of enzymes for the hydrolysis of the protein is a known procedure. The enzymes are usually mixed with the protein to form the protein hydrolysates in a batch procedure. However the use of enzymes in the batch procedure can be prohibitive as the enzymes cannot be collected from the mixture, isolated and reused. Furthermore a cost of the enzymes can be up to 50% of a cost of total raw materials. Therefore the batch procedure for the hydrolysis of proteins has its drawbacks.

Ultra filtration (UF) is a process of separating small molecules such as amino acids and peptides from protein hydrolysate mixtures using membranes. A basis for the separation is size exclusion of molecules such that particles such as amino acids and peptides are retained on the membrane, while other constituents of the mixture such as salt and water pass through the membrane. Therefore UF facilitates amino acid and peptide protein concentration. UF nevertheless has drawbacks and an effectiveness of UF is strongly dependent on operating parameters and hydrolysate characteristics. The operating parameters can be, for example, trans-membrane pressure, membrane cut-off, tangential fluid velocity and system hydrodynamics. The hydrolysate characteristics can be, for example, pH, viscosity, particle size, and salt concentration. That is to say that current UF technology requires a manipulation of a number of factors which is complicated and cumbersome to maintain in order to achieve efficient separation and isolation of the protein hydrolysates.

Plant proteins are partly water-insoluble. A structure of plant proteins is relatively large. A diffusion of plant proteins into an immobilization matrix such as a bed of immobilized enzymes has not been contemplated or considered. Consequently an effectiveness of immobilized enzymes for plant protein hydrolysis is poor.

Nevertheless a use of immobilized proteases for the manufacture of protein hydrolysates from peptides is known. However due to lack of effectiveness the feasibility of such systems is still hindered (see for example Walsh, M., K., Immobilized enzyme for food applications, in Novel enzyme technology for food applications, R. Rastall, Editor. 2007, CRC Press LLC: Boca Raton. p. 60-84).

There is a need to overcome at least some of the aforementioned problems of the prior art.

### Summary of the invention

In a first aspect the present invention relates to a membrane reactor for the manufacture of plant protein hydrolysates. The membrane reactor comprises a substrate vessel adapted to provide a plant protein substrate to an enzyme source. The membrane reactor further comprises a continuously stirred reactor comprising the enzyme source. The membrane reactor further comprises an ultra filtration module comprising a membrane with a molecular cut off wherein the membrane is adapted to allow passage of the plant protein hydrolysates whilst retaining the enzyme.

In a further aspect the present invention relates to a use of the membrane reactor in the manufacture of plant protein hydrolysates for food stuffs.

In a further aspect the present invention relates to a method for the manufacture of plant protein hydrolysates for use in food. The method comprises a first step of providing a suspension of plant protein. To the suspension of plant protein an enzyme is added to form a mixture such that plant protein hydrolysis occurs. Filtering the resulting mixture through an ultra filtration module comprising a membrane with a molecular cut off (e.g. 10 kDa); and finally collecting the filtrate comprising the plant protein hydrolysate for use as a food.

### Brief description of drawings

Figure 1 shows a schematic representation of an operating window for development of membrane reactor technology according to an aspect of the present invention.
Figure 2 shows a diagram of an exemplary set-up for enzymatic hydrolysis of plant proteins with a membrane reactor according to an aspect of the invention.
Figure 3 shows relative enzyme activity [%] in fractions collected during testing of a ceramic membrane in a cross-flow-filtration module membrane with a 5 nanometre cut off with plant protein.
Figure 4 shows temperature stability of glutaminase activity determined with 1-γ-Glutamyl-p-Nitroanilid hydrolysis assay (T 57 ± 1°C and pH 5.0 ± 0.2).
Figure 5 shows temperature stability of protease activity during hydrolysis of wheat gluten over time determined with 1-leucine-para-Nitroanalid assay in the presence of substrate (T 57 ± 1°C and pH 5.0 ± 0.2)
Figure 6 shows plant protein hydrolysate yield over time [g/L*h] in a lab-scale enzyme membrane reactor experiments using 10 kDa, 5 kDa and 1 kDa molecular cut-off membranes according to aspects of the present invention.
Figure 7 shows increased release of amino acids from an enzyme membrane reactor compared to a batch reactor applying the same enzyme concentration and the same size of membrane reactor (50L). (pH 5.0, T 50°C)
Figure 8 shows an amino acid profile of plant protein hydrolysate. The plant protein hydrolysates of the present invention are a naturally balanced mixture of peptides and amino acids.
Figure 9 shows a HPLC analysis that the plant protein hydrolysate of the membrane reactor according to the invention does not differ significantly from plant protein hydrolysate of the batch process.

### Detailed description of invention

For a complete understanding of the present invention and the advantages thereof, reference is made to the following detailed description of the invention taken in conjunction with the accompanying figures.

It should be appreciated that various aspects of the present invention are merely illustrative of the specific ways to make and use the present invention. The various aspects of the present invention can be combined with other aspect of the present invention and do not limit the scope of the invention when taken into consideration with the claims and the following detailed description.

In a first aspect the present invention relates to a membrane reactor for a hydrolysis of a plant protein to form plant protein hydrolysates. The membrane reactor combines advantages of enzyme immobilization (e.g. lower enzyme substrate ratio) and the enzyme batch system (e.g. good enzyme/substrate contact). The Membrane reactor enables large scale hydrolysis of plant proteins to form plant protein hydrolysates.

The membrane reactor increases efficiency of plant protein hydrolysis. The efficiency is increased by re-usage of the enzyme's catalytic activity resulting in a better enzyme/plant protein ratio. Additionally, the removal of plant protein hydrolysate shifts equilibrium of enzymatic action or microbial fermentation towards plant protein hydrolysate. Efficiency of plant protein hydrolysis is thus defined by the following three factors:
● Space-time-yield [g/L/h]
● Enzyme/plant protein hydrolysate ratio [nkat/g]
● Plant protein/Plant protein hydrolysate ratio [%-w/w]

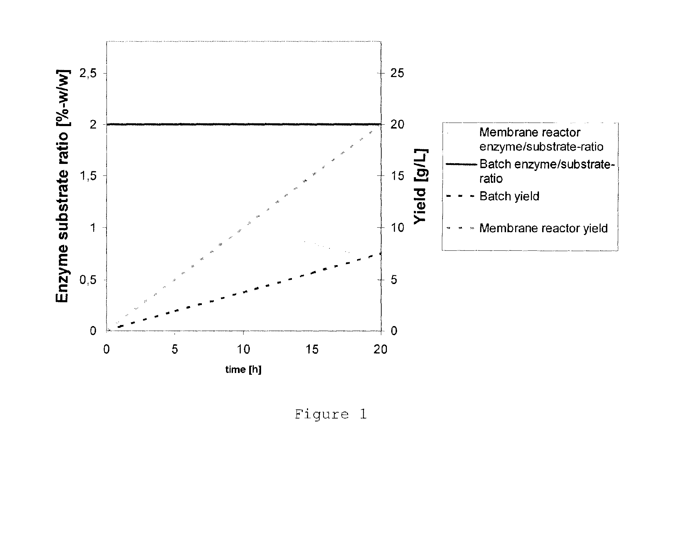

Therefore process efficiency values of the batch process means that an operating window for a semi-continuous membrane bioreactor system can be defined, from which the technological targets for the membrane reactor can be deduced. Figure 1 represents the operating window for the development of a semi-continuous membrane bioreactor system, which is limited to 20 hours due to microbial stability of the enzymes. Furthermore figure 1 shows a schematic representation of the operating window for the development of the membrane reactor technology to enzymatically hydrolyze the plant protein wheat gluten. Thus from Figure 1 it is determined that the enzyme : plant protein ratio (left y-axis, continuous line) must be below 2 %-w/w, as for example represented by the curved continuous line for a typical membrane bioreactor curve, which in this case goes through the break-even point at 6 hours. The space yield over time (right y-axis) must be above the lower dotted line, as for example shown by the upper dotted line.

The operating window as determined from figure 1 was a starting point for setting the experimental parameters in order to test feasibility of a method for manufacturing plant protein hydrolysates using the membrane reactor.

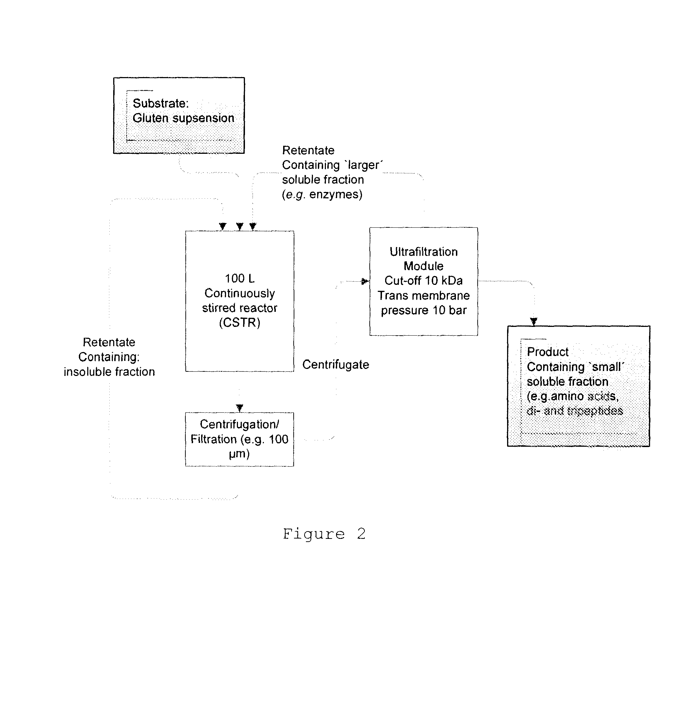

A schematic of an exemplary embodiment of the membrane reactor is shown in figure 2. In figure 2 a vessel for holding the substrate is shown. The substrate is a suspension of a plant protein, for example wheat gluten. The plant protein substrate is then fed to a continuously stirred reactor (CSTR) in which is also present the enzymes to form a mixture. The continuously stirred reactor ensures a homogenous mixture of suspension of the plant protein and enzyme and therefore provides optimal conditions for hydrolysis of the plant protein to form the plant protein hydrolysates. Following hydrolysis, a separation of solid matter and liquid matter may be carried out. Any solid matter following the separation of solid matter and liquid matter is then returned to the continuously stirred reactor. The resulting mixture containing the plant protein hydrolysate is then sent to an ultra filtration module. The ultra filtration module has a membrane with a molecular cut off (MCO). The membrane with the MCO determines which plant protein hydrolysates pass through the membrane. Different membranes can therefore be used. The ultra filtration module has a trans-membrane pressure (TMP) of 10bar. The plant protein hydrolysates such as amino acids and peptides pass through the UF module and can be collected. A retentate that does not pass through the UF module is returned to the continuous stirred reactor and the process repeats. It is to be understood that the membrane reactor is not a closed system and can be continuously replenished with more materials to form the plant protein hydrolysates.

An advantage of having separation of solid matter and liquid matter from the mixture from the continuously stirred reactor prior to filtration is to avoid insoluble matter to foul and enter the membrane of the ultra filtration module. The separation of solid matter and liquid matter decreases the risk of fouling of the membrane and increases the output of plant protein hydrolysate. The of separation of solid matter and liquid matter can be achieved by for example, but not limited to separation techniques such as centrifugation and metal edge filters as known in the art.

The membrane reactor can also include an electro dialysis system (not shown). The electro dialysis system operates by applying electrical potential difference through the membrane such that an electrical charge is passed over the membrane to cause diffusion of polar molecules such as amino acids through the membrane. The electro dialysis system enables a separation of the amino acids and the peptides from the plant protein hydrolysates.

According to an aspect of the present invention the plant protein wheat gluten was mixed with water to obtain a suspension of plant protein of between 0.5 to 50% (w/w), preferably between 0.5% (w/w) to 22%, more preferably between 5 to 10% (w/w). It is observed that when the suspension of plant protein is between 0.5% (w/w) to 22% there is an improvement of pumping properties and a reduction of membrane fouling. In order to maintain a stability of enzyme action a pH of the plant protein in water suspension is adjusted to pH 5 by an addition of acetic acid. Alternatively to maintain a stability of enzyme action the plant protein in water suspension is heated. Heating the plant protein in water suspension is preferred since the heating provides improved accessibility of the plant protein with the enzyme and enables a higher enzyme activity and microbial stability of the enzyme. The wheat gluten suspension is transferred to the continuously stirred reactor with a rate equal to a rate of formation of plant protein hydrolysate - to ensure a continuous manufacture of plant protein hydrolysate. In the continuously stirred reactor, the enzyme (or mixture of enzymes) 20-5000 nkat/L is present for hydrolysis of the plant protein to peptides and amino acids. The mixture entering the ultra filtration module is in cross-flow mode, circulated over a membrane (e.g. ceramic membrane) with a channel size that is large enough to avoid channel blockage by particles that are present in the mixture. A pore-size of the membrane of the ultra filtration module must be small enough to retain enzyme and plant proteins, but large enough to allow protein hydrolysates to pass through the membrane.

It is to be appreciated that following the manufacture of the plant protein hydrolysate. The plant protein hydrolysate can be dried.

The plant protein hydrolysates are useful for providing taste active ingredients to food products.

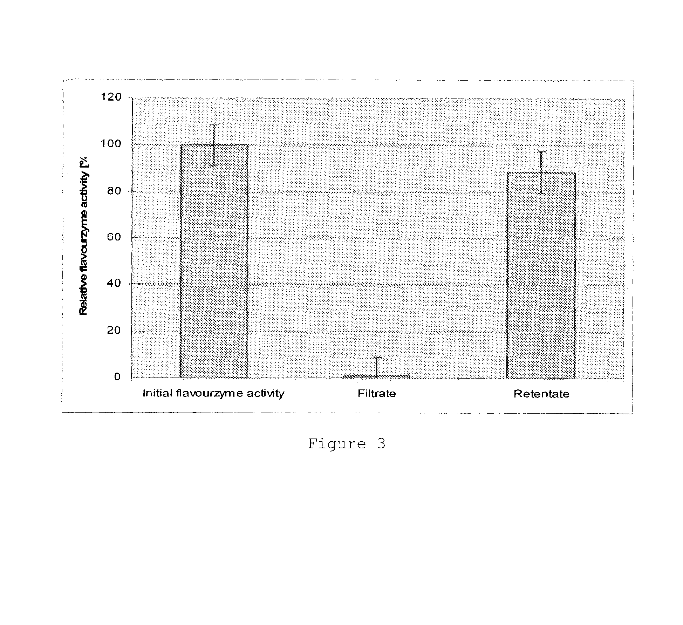

A ceramic membrane of the ultra filtration device with a 5 nanometre molecular cut off pore-size was tested for enzyme retention with plant protein. In various aspects of the present invention the membrane of the ultra filtration device can have a molecular cut off pore-size of between 1 to 20 nanometres. The aim of the test was to assess if a technical protease enzyme cocktail (Flavorzyme, [E] 264 nkat/L Leu-p-Na) passed though the membrane in the presence of plant protein (10 %-w/w wheat gluten). Enzyme retention by the membrane is important for the technical feasibility of the membrane bioreactor for plant protein hydrolysis. The results are shown in figure 3 in which it seen that no significant enzyme activity has been lost over a time period of 3 hours.

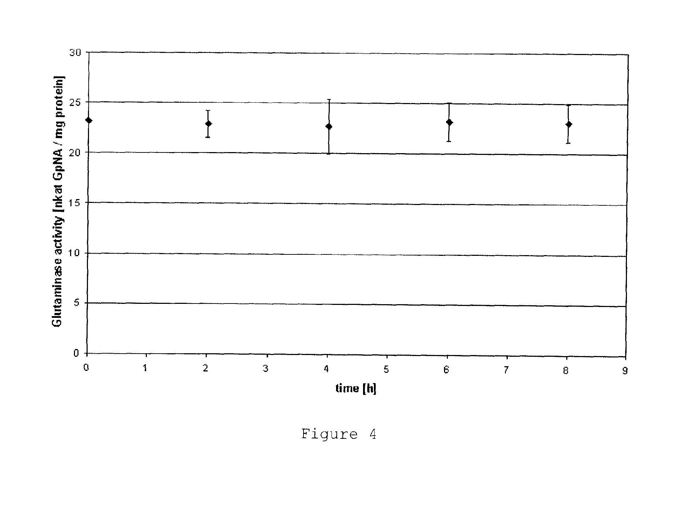

Furthermore a stability of glutaminase activity was followed under process conditions (57 ± 1°C and pH 5.0 ± 0.2 in the presence of substrate) by hydrolysis of the chromogenic substrate L-γ-Glutamyl-p-Nitroanilide (GpNA). The results of this stability investigation are shown in figure 4. Accordingly, glutaminase activity was stable over 8 hours processing time which is in accordance with earlier findings (see Mohamed i. Mahmoud, C.T.C., Protein Hydrolysates as Special Nutritional Ingredients. Novel Macromolecules in Food Systems, 2000: p. 181-215).

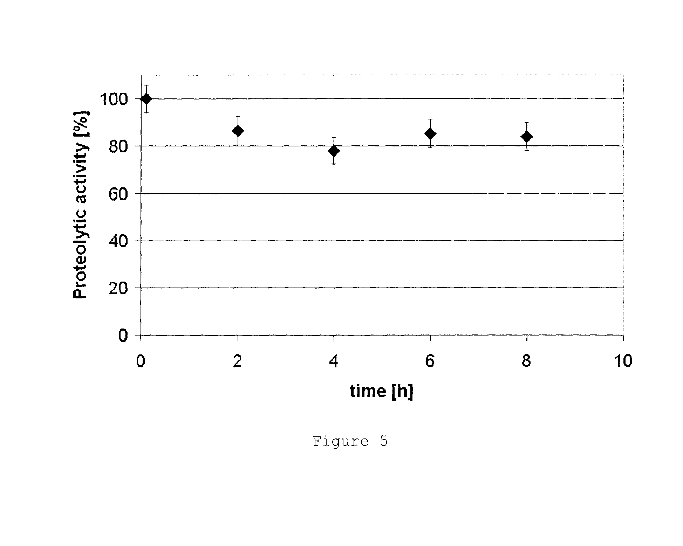

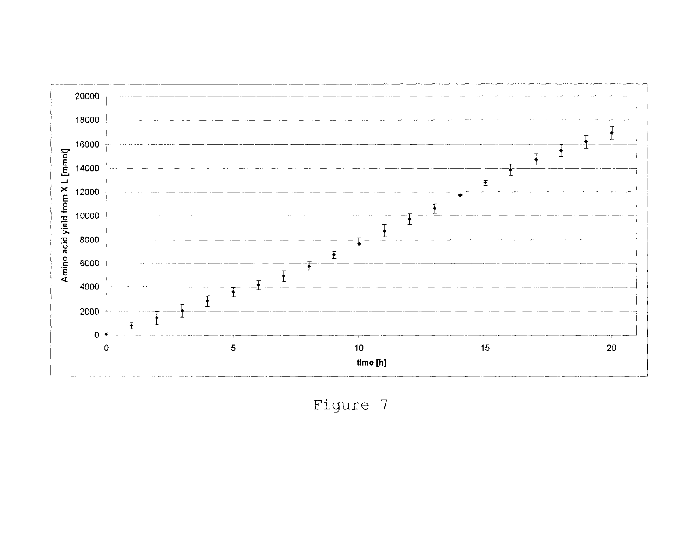

An enzyme activity of the protease enzyme flavorzyme (available from Novozymes A/S) was determined with the leucine para-nitroanilide method and wheat gluten substrate (see Deeslie, M.C.a.W.D., Soy Protein Hydrolysis in Membrane Reactors. JAOCS, 1983. 60(6): pp. 1112-1115). The initial activity was 264 nkat/L. Figure 5 shows the relative activity of the enzyme flavorzyme during 8 hours at 57 ± 1°C and pH 5.0 ± 0.2, measured with the leucine para nitroanilide method. After 24 hours at 57 ± 1 °C the relative flavorzyme activity was still 71 ± 6%, which indicates a loss in enzyme activity per hour of slightly more than 1%. Figure 5 demonstrates that the enzyme activity is stable under process conditions. The cause for a declined reaction rate (triangular line of figure 7) is product inhibition. By removing product via the membrane, product inhibition is evaded and the efficiency of the reactor and enzyme increases.

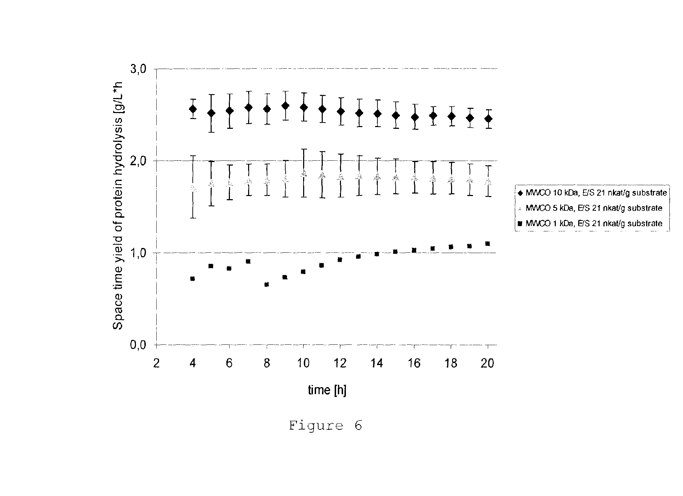

Laboratory tests with a membrane reactor using a 10 kDa filter and a wheat gluten substrate concentration of 0.5 % (w/w) showed proof of principle of the membrane reactor for enzymatic wheat gluten hydrolysis. The product/substrate ratio of the membrane reactor was 51% since the amount of substrate used was 1 gram over 20 hours and the total product yield was 0.51 gram. The batch hydrolysis under the same conditions with the same absolute amount of enzyme (0.89 nkat/g), but a total reaction volume of 50 mL which correlates with a substrate amount of 0.25 gram resulted in a product/substrate ratio of 70% using a molecular weight cut-off (MWCO) of 10 kDa since the product yield was 0.18 gram. Therefore according to the invention the enzyme usage efficiency increased almost 3 times. The results are shown in figure 6. Figure 6 shows in the top curve results using a MWCO of 10KDa and an amount of enzyme/plant protein hydrolysate ratio (21 nkat/g). Figure 6 shows in the middle curve results using a MWCO of 5KDa and an amount of enzyme/plant protein hydrolysate ratio (21 nkat/g). Figure 6 shows in the lower curve results using a MWCO of 1KDa and an amount of enzyme/plant protein hydrolysate ratio (21 nkat/g).

The plant protein of the present invention can of course be derived from other sources of plant protein aside from whey. The sources of plant protein can include, but are not limited to plant protein derived from, soy, corn, potato, pea or cassava.

The enzymes of the present invention can be a single enzyme or a mixture of enzymes. The enzyme can be enzyme is at least one of an endopeptidase, an exopeptidase a glutaminsae and an enzyme derived from Basidiomycetes.

Also at pilot plant scale technical feasibility was evaluated. A significant improvement of amino acid release over time was shown when applying the same size of membrane reactor and the same enzyme concentration as shown in figure 7. Further optimization of operational conditions will result in even better amino acids yields.

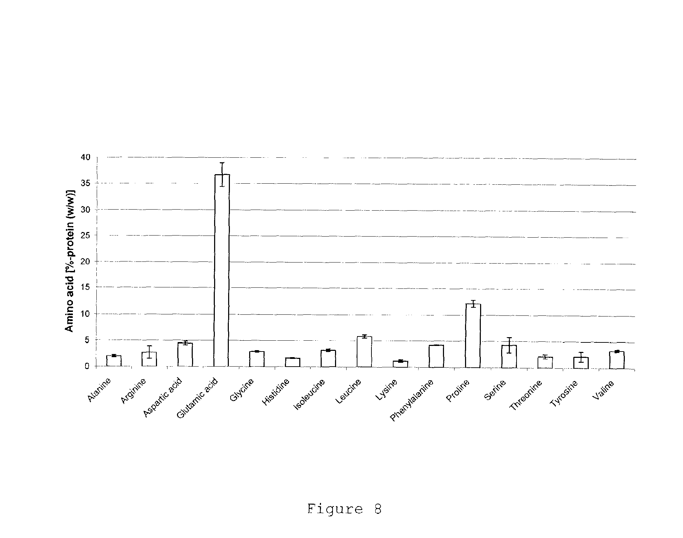

The amino acid profile of the plant protein hydrolysate is shown in figure 8. The amino acid profile of the plant protein hydrolysate was determined to comprise no residual plant protein, at least 10 percent of the peptides of the plant protein hydrolysate containing 2 to 5 amino acids, the amount of free amino acids being higher than 30 percent.

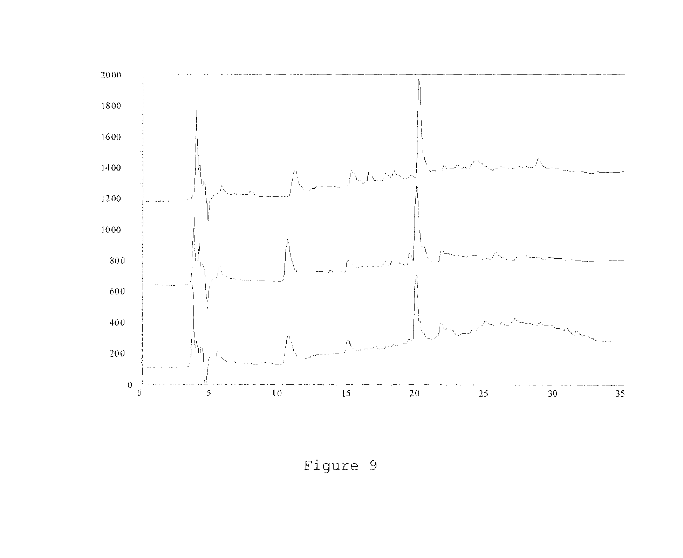

As shown in figure 9, HPLC analysis shows that the plant protein hydrolysate does not differ significantly from plant protein hydrolysate of the batch process. The top line of figure 9 shows filtered wheat gluten hydrolysis product from factory production sample (0.45 µm filtered). The middle line shows batch produced wheat gluten hydrolysis product which was produced for comparison at bench scale (10kDa filtered). The bottom line shows a ten hour sample from the membrane reactor experiment using a 10 kDa Molecular Weight Cut-Off (MWCO) membrane.

The invention includes the combination of state of the art science and technology on powder wetting, enzyme kinetic understanding (biotransformation), membrane bioreactor technology (Fractionation and Membrane Technology), sensory analysis, and understanding recent trends in consumer market trends research and product application.

The present invention provides energy efficiency and operational simplicity, high transport selectivity, large operational flexibility and environment compatibility. The invention provides means for enhanced molecular separations and chemical transformations overcoming existing limits of the traditional industrial processes.

The advantages of the present invention demonstrate that the enzymes applied are still active at the end of the plant protein hydrolysis. In the batch process the enzymes need to be inactivated at the end of the protein hydrolysis. Since the enzymatic (also called bio-catalytic) function is catalytic it is logical that a more efficient use of enzyme can be obtained by retaining or recovering it during or after plant protein hydrolysis.

The present invention enables the fractionation and concentration of plant protein hydrolysates according to size. An advantage of plant protein hydrolysates is the perception by the consumer especially in the case of the vegetarian consumer who would prefer not to consume animal derived protein hydrolysates. Furthermore the plant protein hydrolysates are manufactures in a natural way.

Having thus described the present invention in detail, it is to be understood that the detailed description is not intended to limit the scope of the invention thereof.

What is desired to be protected by letters patent is set forth in the following claims.

## Claims

1. A membrane reactor for the manufacture of plant protein hydrolysates, the membrane reactor comprising:
- a substrate vessel adapted to provide a plant protein substrate to an enzyme source,
- a continuously stirred reactor comprising the enzyme source; and
- an ultra filtration module comprising a membrane with a molecular cut off wherein the membrane is adapted to allow passage of the plant protein hydrolysate whilst retaining the enzyme.

2. The membrane reactor of claim 1 further comprising a heating device adapted to maintain a temperature of the content of the continuously stirred reactor between 25°C and 75°C.

3. The membrane reactor of any one of the above claims further comprising an electro dialysis system.

4. The membrane reactor of any one of the above claims further comprising a separation device, capable of separating insoluble matter from the plant protein hydrolysate.

5. The membrane reactor of any one of the above claims wherein the membrane has a pore size of between 1 to 20 nanometres, preferably 5 nanometres.

6. The membrane reactor of any one of the above claims, enzyme is at least one of an endopeptidase, an exopeptidase, a glutaminsae or an enzyme derived from Basidiomycetes.

7. A use of the membrane reactor of any one of the preceding claims in the manufacture of plant protein hydrolysates for food stuffs.

8. A method for the manufacture of plant protein hydrolysates for use in food, the method comprising:
- providing a suspension of plant protein,
- adding to the suspension of plant protein an enzyme to form a mixture such that plant protein hydrolysis occurs,
- filtering the resulting mixture through an ultra filtration module comprising a membrane with a molecular cut off; and
- Collecting the filtrate comprising the plant protein hydrolysate for use as a food.

9. The method according to claim 8, wherein the suspension of plant protein comprises between 0.5 to 50% (w/w) plant protein.

10. The method according to any of claims 8 to 9, wherein a source of the plant protein is selected from wheat, soy, corn, potato, pea or cassava.

11. The method according to any of claims 8 to 10, wherein the enzyme is at least one of an endopeptidase, an exopeptidase, a glutaminase or an enzyme derived from Basidiomycetes.

12. The method according to any of claims 8 to 11, wherein a pH of the mixture is adjusted.

13. The method according to any of claims 8 to 12, wherein a temperature of the mixture is adjusted.

14. The method according to any of claims 8 to 13, wherein prior to the filtration, insoluble material is separated from the mixture.

15. The method according to claim 14, wherein the insoluble material is separated from the mixture by any one of a centrifuge and a metal edge filter.

16. A plant protein hydrolysate obtainable by the method of any one of claims 8 to 15, wherein the plant protein hydrolysates are taste enhancing compounds for use in foodstuffs.
